# EUROPÄISCHE PATENTSCHRIFT

(11) **EP 2 197 526 B1**
(45) Veröffentlichungstag und Bekanntmachung des Hinweises auf die Patenterteilung: **25.07.2018**
(21) Anmeldenummer: 08804679.2
(22) Anmeldetag: 24.09.2008
(51) Int. Cl.: A62B 7/14, A61M 16/10, B64D 10/00

(54) **SYSTEM ZUM BEREITSTELLEN VON NOTFALLSAUERSTOFF SOWIE THERAPEUTISCHEM SAUERSTOFF**
SYSTEM FOR PROVIDING EMERGENCY OXYGEN AND THERAPEUTIC OXYGEN
SYSTÈME DESTINÉ À FOURNIR DE L'OXYGÈNE D'URGENCE AINSI QUE DE L'OXYGÈNE THÉRAPEUTIQUE

(30) Priorität: 12.10.2007 DE 102007048924; 12.10.2007 US 998781 P
(43) Veröffentlichungstag der Anmeldung: 23.06.2010
(73) Patentinhaber: Airbus Operations GmbH, 21129 Hamburg (DE)
(72) Erfinder: VOGT, Thomas, 22547 Hamburg (DE); WESTPHAL, Andreas, 23701 Eutin (DE); DEGENHARDT, Detlev, 23617 Stockelsdorf (DE)
(74) Vertreter: LKGLOBAL Lorenz & Kopf PartG mbB Patentanwälte
(86) Internationale Anmeldenummer: PCT/EP2008/062774
(87) Internationale Veröffentlichungsnummer: WO 2009/050006

(56) Entgegenhaltungen:
- EP-A- 1 512 433
- WO-A-96/16718
- FR-A- 2 874 509
- US-A- 3 045 691
- US-A1- 2003 233 936

## Beschreibung

Die Erfindung betrifft ein System zum Bereitstellen von Notfallsauerstoff sowie therapeutischem Sauerstoff in einem Flugzeug, eine Vorrichtung zum Bereitstellen von therapeutischem Sauerstoff, ein Flugzeug aufweisend ein System zum Bereitstellen von Notfallsauerstoff sowie therapeutischem Sauerstoff und die Verwendung eines Systems zum Bereitstellen von Notfallsauerstoff sowie therapeutischem Sauerstoff in einem Flugzeug.

Herkömmliche Vorrichtungen zum Bereitstellen von therapeutischem Sauerstoff sind nur an einzelnen Stellen im Flugzeug vorgesehen, so überhaupt vorhanden, und sind auch meist als zusätzliche, nicht integrierte Komponenten ausgebildet. Herkömmliche Vorrichtungen zum Bereitstellen von therapeutischem Sauerstoff weisen damit eine stark eingeschränkte Funktionalität bzw. Flexibilität auf.

US 2003/233936 A1 beschreibt einen Sauerstoff- und Inertgasgenerator, der in Luftfahrzeugen verwendbar ist, und insbesondere ein hybrides System zum Erzeugen von Sauerstoff an Bord und ein System zum Erzeugen von Inertgas an Bord.

Es ist eine Aufgabe der Erfindung, eine Vorrichtung zum Bereitstellen von Notfallsauerstoff sowie therapeutischem Sauerstoff aufzuzeigen, welche über eine erhöhte Flexibilität und Benutzerfreundlichkeit verfügt.

Dieses Problem wird durch ein System zum Bereitstellen von Notfallsauerstoff sowie therapeutischem Sauerstoff, durch eine Vorrichtung zum Bereitstellen von therapeutischem Sauerstoff, ein Flugzeug aufweisend ein System zum Bereitstellen von Notfallsauerstoff sowie therapeutischem Sauerstoff und die Verwendung eines Systems zum Bereitstellen von Notfallsauerstoff sowie therapeutischem Sauerstoff in einem Flugzeug gelöst.

Gemäß einem exemplarischen Ausführungsbeispiel der Erfindung ist ein System zum Bereitstellen von Notfallsauerstoff sowie therapeutischem Sauerstoff in einem Flugzeug geschaffen, aufweisend eine Sauerstoffquelle, ein Sauerstoffleitungssystem mit einem zweiten Sauerstoffausgang, eine Vorrichtung zum Bereitstellen von Notfallsauerstoff mit einem zweiten Sauerstoffeingang, eine Vorrichtung zum Bereitstellen von therapeutischem Sauerstoff wie unten beschreiben, wobei der zweite Sauerstoffausgang mit dem ersten Sauerstoffeingang koppelbar ist, der erste Sauerstoffausgang mit dem zweiten Sauerstoffeingang koppelbar ist und der zweite Sauerstoffausgang mit dem zweiten Sauerstoffeingang koppelbar ist.

Gemäß einem weiteren exemplarischen Ausführungsbeispiel der Erfindung ist eine Vorrichtung zum Bereitstellen von therapeutischem Sauerstoff geschaffen, aufweisend einen ersten Sauerstoffeingang und einen ersten Sauerstoffausgang, wobei ein Sauerstoffausgang eines Sauerstoffleitungssystems mit dem ersten Sauerstoffeingang koppelbar ist, der erste Sauerstoffausgang mit einem Sauerstoffeingang einer Vorrichtung zum Bereitstellen von Notfallsauerstoff koppelbar ist, wobei der erste Sauerstoffeingang intern zu dem ersten Sauerstoffausgang durchgeschleift ist, wobei die Vorrichtung zum Bereitstellen von therapeutischem Sauerstoff einen ersten Anschluss für eine Applikationsvorrichtung aufweist, wobei der erste Anschluss für eine erste vorbestimmte Durchflussrate ausgelegt ist, und wobei die Vorrichtung zum Bereitstellen von therapeutischem Sauerstoff ferner einen zweiten Anschluss für eine Applikationsvorrichtung aufweist, wobei der zweite Anschluss für eine zweite vorbestimmte Durchflussrate ausgelegt ist, die sich von der ersten vorbestimmten Durchflussrate unterscheidet.

Gemäß einem weiteren exemplarischen Ausführungsbeispiel der Erfindung ist ein Flugzeug geschaffen, aufweisend ein System zum Bereitstellen von Notfallsauerstoff sowie therapeutischem Sauerstoff mit den oben beschriebenen Merkmalen.

Gemäß einem weiteren exemplarischen Ausführungsbeispiel der Erfindung wird ein System zum Bereitstellen von Notfallsauerstoff sowie therapeutischem Sauerstoff mit den zuvor beschriebenen Merkmalen in einem Flugzeug verwendet.

Im Rahmen der vorliegenden Patentanmeldung werden folgende Definitionen verwendet:

### Notfallsauerstoff:

Notfallsauerstoff findet im Flugzeug in Situationen Verwendung, in denen eine ausreichende Sauerstoffversorgung der Passagiere durch einfaches Atmen der Kabinenluft nicht mehr sichergestellt sein mag. Dies mag charakterisiert sein durch einen über einen im normalen Flugbetrieb herrschenden Kabinendruck weiter reduzierten Kabinendruck, zum Beispiel aufgrund eines Lecks oder Risses in der Flugzeughülle, so dass der zur Verfügung stehende Sauerstoffpartialdruck der Kabinenluft für eine ausreichende Sauerstoffversorgung des Passagiers nicht mehr ausreichend sein mag. In solch einem Fall erfolgt die Applikation von Notfallsauerstoff, zum Beispiel durch Bereitstellen durch automatisches Auslösen der bekannten Sauerstoffmasken aus der Kabinendecke bzw. dem Personal Supply Channel des Flugzeugs. Unter Verwendung dieser Notfall-Sauerstoffmasken wird den Passagieren individuell Sauerstoff zur Verfügung gestellt, um so insgesamt eine ausreichende Sauerstoffversorgung des Körpers des jeweiligen Passagiers sicherzustellen.

### Therapeutischer Sauerstoff:

Therapeutischer Sauerstoff unterscheidet sich in seinen physikalischen Eigenschaften, wie zum Beispiel Temperatur und Wassersättigung, nicht vom Notfallsauerstoff. Somit mag der therapeutische Sauerstoff auch von der gleichen Sauerstoffquelle bereitgestellt werden wie der Notfallsauerstoff. Da der therapeutische Sauerstoff im regulären Betriebsfall bereitgestellt wird, somit unter regulären Druckbedingungen in der Kabine, mag der Druck im Sauerstoff-Leitungssystem des Flugzeugs und damit die Durchflussrate von therapeutischem Sauerstoff geringer sein als die Durchflussrate von Notfallsauerstoff. Therapeutischer Sauerstoff wird meist aufgrund einer medizinischen Indikation dem jeweiligen Passagier, nach Möglichkeit permanent während des gesamten Fluges, zur Verfügung gestellt, um diesem beispielsweise das Atmen zu erleichtern, oder aufgrund einer weiteren medizinischen Indikation ein erhöhtes Sauerstoffangebot darzubieten.

### Sauerstoffquelle:

Sauerstoffquelle ist jede Vorrichtung, die geeignet ist, reinen bzw. hochprozentigen Sauerstoff in einem Flugzeug bereitzustellen. Mögliche Sauerstoffquellen sind hier beispielsweise chemische Sauerstoffgeneratoren, tragbare bzw. fest installierte Sauerstoffflaschen oder auch tragbare bzw. fest installierte Sauerstoffgeneratoren, sogenannte OBOGS.

### Betriebszustände:

In Rahmen der vorliegenden Beschreibung der Erfindung finden sich Verweise auf zwei unterschiedliche Betriebszustände.

Der erste Betriebszustand ist hierbei charakterisiert durch das Betreitstellen von Notfallsauerstoff unter Verwendung der bekannten Notfall-Sauerstoffmasken (oder, im Falle, dass zuvor einem Passagier therapeutischer Sauerstoff bereitgestellt wurde, auch durch die hierzu verwendete Applikationsvorrichtung für therapeutischen Sauerstoff) wie zuvor angezeigt beispielsweise im Falle eines Druckverlusts der Flugzeugkabine. Der erste Betriebszustand stellt damit regelmäßig eine außergewöhnliche Notfallsituation im Flugbetrieb dar.

Der zweite Betriebszustand ist charakterisiert durch das Bereitstellen von therapeutischem Sauerstoff unter Verwendung einer Applikationsvorrichtung, beispielsweise einer medizinischen Sauerstoffmaske. Der zweite Betriebszustand stellt damit keinen außergewöhnlichen Betriebszustand des Flugbetriebs dar, sondern wird auf Wunsch des Passagiers bzw. aufgrund einer medizinischen Indikation während des regulären Flugbetriebs angeboten.

Anschaulich ist gemäß einem exemplarischen Ausführungsbeispiel der Erfindung ein System zum Bereitstellen von Notfallsauerstoff sowie therapeutischem Sauerstoff in einem Flugzeug geschaffen, das es ermöglicht, flexibel und individuell an einer Vielzahl oder an allen möglichen Sitzplätzen im Flugzeug sowohl Notfallsauerstoff als auch therapeutischen Sauerstoff zur Verfügung zu stellen.

Durch die Verwendung einer gemeinsamen Sauerstoffquelle für das Bereitstellen von Notfallsauerstoff sowie das Bereitstellen von therapeutischem Sauerstoff und insbesondere durch die Verwendung desselben Sauerstoffleitungssystems im Flugzeug lässt sich das erfindungsgemäße System wesentlich einfacher aufbauen bzw. mag dieses sogar nachträglich leicht umrüstbar bzw. erweiterbar sein.

Hierbei mag das erfindungsgemäße System das Bereitstellen von therapeutischem Sauerstoff an jedem beliebigen Sitzplatz im Flugzeug ermöglichen, wodurch logistische Probleme bei der Sitzplatzvergabe nicht mehr auftreten mögen, eine spezielle Berücksichtigung des Erfordernisses von therapeutischem Sauerstoff mag somit beim Check-In nicht mehr notwendig sein.

Eine Integration der Vorrichtung zum Bereitstellen von therapeutischem Sauerstoff in den Personal Supply Channel (PSC) eines Flugzeuges mag darüber hinaus die optisch nicht invasive Integration in das Flugzeug ermöglichen, wodurch ein harmonischer Gesamteindruck der Flugzeugkabine erzielt bzw. dieser nicht unnötig gestört werden mag.

Somit mag das erfindungsgemäße System modular ausgebildet sein, um, je nach Anforderung der Airline, bei der Erstausstattung oder aber auch flugindividuell, eine Integration für die Vorrichtung zum Bereitstellen von therapeutischem Sauerstoff zu ermöglichen. Es mag auch entschieden werden, nur wenige Vorrichtungen zum Bereitstellen von therapeutischem Sauerstoff anzubringen, welche sich je nach Bedarf und aktueller Anforderung umrüsten bzw. anordnen lassen.

Weitere exemplarische Ausgestaltungen ergeben sich aus den anhängigen Ansprüchen.

Im Weiteren werden Ausgestaltungen des erfindungsgemäßen Systems zum Bereitstellen von Notfallsauerstoff sowie therapeutischem Sauerstoff beschrieben. Diese Ausgestaltungen gelten allerdings ebenso für die Vorrichtung zum Bereitstellen von therapeutischem Sauerstoff, für das Flugzeug aufweisend ein System zum Bereitstellen von Notfallsauerstoff und therapeutischem Sauerstoff sowie für die Verwendung eines Systems zum Bereitstellen von Notfallsauerstoff sowie therapeutischem Sauerstoff in einem Flugzeug.

Nach einem weiteren exemplarischen Ausführungsbeispiel mag das System zum Bereitstellen von Notfallsauerstoff und therapeutischem Sauerstoff weiterhin an der Vorrichtung zum Bereitstellen von therapeutischem Sauerstoff einen ersten Anschluss für eine Applikationsvorrichtung aufweisen, wobei der erste Anschluss für eine vorbestimmte Durchflussrate ausgelegt ist.

Durch einen entsprechenden Anschluss für eine Applikationsvorrichtung, zum Beispiel eine medizinische Sauerstoffmaske oder auch eine reguläre Sauerstoffmaske, wie sie im Rahmen des Bereitstellens von Notfallsauerstoff im Flugzeug verwendet wird, mag eine einfache Applikation des therapeutischen Sauerstoffs an den bedürftigen Passagier möglich sein.

Das Verwenden eines ersten Anschlusses mit einer ersten vorbestimmten Durchflussrate erhöht die Einfachheit und Benutzerfreundlichkeit des erfindungsgemäßen Systems weiter, da eine individuelle Einstellung der Durchflussrate nicht nötig und somit eine mögliche Fehlerquelle ausgeschlossen sein mag.

Die Durchflussrate mag hierbei abhängig von einem bestimmten Druck im Sauerstoffleitungssystem während der Applikation von therapeutischem Sauerstoff sein oder aber mag unabhängig von dem Druck im Sauerstoffleitungssystem im Wesentlichen immer eine vorbestimmte Durchflussrate bereitstellen.

Nach dem exemplarischen Ausführungsbeispiel des erfindungsgemäßen Systems zum Bereitstellen von Notfallsauerstoff sowie therapeutischem Sauerstoff weist die Vorrichtung zum Bereitstellen von therapeutischem Sauerstoff einen zweiten Anschluss für eine Applikationsvorrichtung auf, wobei der zweite Anschluss für eine zweite vorbestimmte Durchflussrate ausgelegt sein ist. Durch den zweiten Anschluss wirdes ermöglicht, in derselben Sitzreihe einen zweiten Passagier unproblematisch mit therapeutischem Sauerstoff zu versorgen. Die zweite vorbestimmte Durchflussrate ist von der ersten vorbestimmten Durchflussrate unterschiedlich. Somit ist es möglich, zwei Passagiere mit unterschiedlichen Durchflussraten wie zum Beispiel einem Passagier mit zwei Litern und einem zweiten Passagier mit vier Litern Sauerstoff pro Minute zu versorgen, oder aber es mag möglich sein, einem einzelnen Passagier individuell, von seinem jeweiligen Zustand, Bedürfnissen oder Wünschen abhängig, eine aktuell angezeigte und/oder medizinisch indizierte Menge an therapeutischem Sauerstoff zur Verfügung zu stellen.

Nach einem weiteren exemplarischen Ausführungsbeispiel des erfindungsgemäßen Systems zum Bereitstellen von Notfallsauerstoff sowie therapeutischem Sauerstoff mag zumindest einer des ersten und des zweiten Anschlusses autoaktivierend ausgebildet sein.

Ein autoaktivierender Anschluss mag hierbei durch einfaches Einstecken eines Teils der Applikationsvorrichtung, wie zum Beispiel eines Schlauchs, einen Sauerstofffluss ermöglichen, ohne dass dieser separat am Anschluss oder an der Vorrichtung aktiviert worden sein mag.

Ein autoaktiviercnder Anschluss mag somit die notwendigen einzelnen Schritte zum Bereitstellen von therapeutischem Sauerstoff reduzieren bzw. minimieren. Eine mögliche Fehlerquelle des Nichtaktivierens eines Anschlusses beim Bereitstellen von therapeutischem Sauerstoff mag somit ausgeschlossen sein.

Nach einem weiteren exemplarischen Ausführungsbeispiel des erfindungsgemäßen Systems zum Bereitstellen von Notfallsauerstoff sowie therapeutischem Sauerstoff mag zumindest eine der Vorrichtungen zum Bereitstellen von Notfallsauerstoff und zum Bereitstellen von therapeutischem Sauerstoff eingerichtet sein, in einem Personal Supply Channel eines Flugzeuges eingebracht zu werden.

Durch ein entsprechendes Einbringen in den PSC mag es ermöglicht werden, eine entsprechende Vorrichtung zum Bereitstellen von therapeutischem Sauerstoff für jede Sitzreihe individuell anzubieten.

Durch das Einbringen in den PSC mag es möglich sein, die Vorrichtung zum Bereitstellen von therapeutischem Sauerstoff derart in die Flugzeugsysteme zu integrieren, so dass diese Vorrichtung optisch nicht invasiv in das Flugzeug integriert sein mag. Durch einen versenkten Einbau in den PSC und unter Verwendung beispielsweise einer Deckklappe mag die Vorrichtung zum Bereitstellen von therapeutischem Sauerstoff im nichtbenötigten Fall komplett verschwinden, damit nicht sichtbar sein.

Somit mag es wirkungsvoll vermieden werden, dass Passagiere beispielsweise von einer offen stehenden und damit sichtbaren Vorrichtung beunruhigt werden, sich aus Unachtsamkeit daran verletzen. Weiterhin mag den Passagieren auch die Möglichkeit genommen werden die Vorrichtung selbstständig zu betätigen und damit im schlimmsten Fall zu beschädigen.

Auch mag es denkbar sein, eine erfindungsgemäße Vorrichtung zum Bereitstellen von therapeutischem Sauerstoff mittels Schnellmontagekopplungen und Schnellkoppelelementen am Sauerstoffeingang bzw. am Sauerstoffausgang kurzfristig, zum Beispiel während der üblichen Standzeit eines Flugzeugs zwischen zwei Flügen, individuell in den PSC und somit in das Flugzeug einzubringen. Ein solches Einbringen einer Vorrichtung zum Bereitstellen von therapeutischem Sauerstoff mag beispielsweise einen vorbestimmten, jedoch nicht belegten und somit möglicherweise nur mit einer Blindklappe versehenen Platz im PSC benützen. Hierdurch mag die Möglichkeit gegeben sein, eine erfindungsgemäße Vorrichtung zum Bereitstellen von therapeutischem Sauerstoff in jeder beliebigen Sitzplatzreihe in einem Flugzeug anzubringen, ohne dies jedoch bereits beim erstmaligen Ausstatten des Flugzeuges vornehmen zu müssen. Somit mag nachträglich eine Individualisierung der Flugzeuginnenausstattung möglich sein, oder aber diese mag auch nur auf eine bestimmte individuelle Situation während eines speziellen gebuchten Fluges anpassbar sein.

Nach einem weiteren exemplarischen Ausführungsbeispiel des erfindungsgemäßen Systems zum Bereitstellen von Notfallsauerstoff sowie therapeutischem Sauerstoff mag der Sauerstoffbetriebsdruck im Sauerstoffleitungssystem in einem zweiten Betriebszustand zur Applikation von therapeutischem Sauerstoff geringer sein als in einem ersten Betriebszustand zur Applikation von Notfallsauerstoff, insbesondere geringer als ein Auslösedruck, mit dem die Vorrichtung zum Bereitstellen von Notfallsauerstoff aktivierbar ist.

Hierdurch mag es ermöglicht werden, die Vorrichtung zum Bereitstellen von therapeutischem Sauerstoff an das gleiche Leitungssystem anzuschließen wie für die Vorrichtung zum Bereitstellen von Notfallsauerstoff verwendet wird. Jedoch mag der Auf- und Einbau der Vorrichtung zum Bereitstellen von therapeutischem Sauerstoff wesentlich vereinfacht werden und mag im Weiteren wesentlich kostengünstiger erfolgen.

Durch einen geringeren Sauerstoffbetriebsdruck im Leitungssystem, beispielsweise 1 bar, im Rahmen des zweiten Betriebszustandes mag außerdem das zufällige und unbeabsichtigte Auslösen der Vorrichtung zum Bereitstellen von Notfallsauerstoff, beispielsweise das Herunterfallen der Notfallsauerstoffmasken, vermieden werden, welche einen wesentlich höheren Sauerstoffbetriebsdruck im Leitungssystem erfordern mag.

Nach einem weiteren exemplarischen Ausführungsbeispiel der vorliegenden Erfindung mag die Vorrichtung zum Bereitstellen von therapeutischem Sauerstoff eine Regelvorrichtung zum Einstellen einer vorbestimmten Sauerstoffflussrate aufweisen.

Durch eine solche Regelvorrichtung mag es möglich sein, die vorbestimmte Sauerstoffflussrate individuell auf die Bedürfnisse eines Passagiers anzupassen, dem therapeutischer Sauerstoff bereitgestellt werden soll.

Auch mag hierdurch beispielsweise ein Druckabfall oder Druckschwankungen im Sauerstoffleitungssystem kompensiert werden. Weiterhin mag denkbar sein, beispielsweise bei der Versorgung von mehreren oder auch einer Vielzahl von Passagieren mit therapeutischem Sauerstoff, auf eine damit veränderte Lastsituation im Sauerstoffleitungssystem zu reagieren und diese zu kompensieren.

Auch mag ein Passagier, dem therapeutischer Sauerstoff bereitgestellt werden soll, selbst individuell die vorbestimmte Sauerstoffflussrate seiner jeweiligen Gesamtverfassung anpassen. Hierbei mag sichergestellt sein, dass eine bestimmte minimale Sauerstoffflussrate (beispielsweise zwei Liter pro Minute) nicht unterschritten sowie eine bestimmte maximale Sauerstoffflussrate (beispielsweise vier oder auch sechs Liter pro Minute) nicht überschritten wird.

Nach einem weiteren exemplarischen Ausführungsbeispiel der vorliegenden Erfindung mag die Vorrichtung zum Bereitstellen von therapeutischem Sauerstoff derart eingerichtet sein, um im ersten Betriebszustand an dem zumindest einen Anschluss eine vorbestimmte Notfallsauerstoffflussrate bereitzustellen.

Somit mag vermieden werden, dass es beispielsweise im Falle eines Druckabfalls in der Kabine nötig werden mag, die Maske zum Bereitstellen von therapeutischem Sauerstoff gegen eine Notfallsauerstoffmaske zu tauschen. Auf diese Weise mag sichergestellt sein, dass der Passagier immer ausreichend mit zusätzlichem Sauerstoff versorgt wird.

Ebenso ist denkbar, dass die vorbestimmte Notfallsauerstoffflussrate, die über die Vorrichtung zum Bereitstellen von therapeutischem Sauerstoff im ersten Betriebszustand abgegeben wird, höher liegt als die reguläre Notfallsauerstoffflussrate, die durch die Vorrichtung zum Bereitstellen von Notfallsauerstoff abgegeben werden würde. Dies mag die auch im Notfall weiter über den normalen Bedarf hinaus erhöhte, benötigte Sauerstoffmenge des Passagiers widerspiegeln.

Nach einem weiteren exemplarischen Ausführungsbeispiel der vorliegenden Erfindung mag das System zum Bereitstellen von Notfallsauerstoff sowie therapeutischem Sauerstoff weiterhin eine Vorrichtung zum Aktivieren bzw. Deaktivieren des zweiten Betriebszustandes aufweisen.

Diese Vorrichtung mag an zentraler bzw. geschützter Stelle im Flugzeug angebracht sein, um so eine Bedienung ausschließlich durch das Flugpersonal sicherzustellen, oder aber sie mag mit der Autoaktivierung eines Anschlusses der Vorrichtung zum Bereitstellen von therapeutischem Sauerstoff gekoppelt sein.

Hierdurch mag vermieden werden, dass ein permanenter Versorgungsdruck für das Bereitstellen von therapeutischem Sauerstoff im Sauerstoffleitungssystem anliegt, ohne dass überhaupt Bedarf hierfür besteht. Somit mag ein unnötiger Sauerstoffverbrauch bzw. ein frühzeitiger Verschleiß des Sauerstoffleitungssystems, zum Beispiel an möglicherweise vorhandenen Dichtungen, vermieden werden.

Im Weiteren werden Ausgestaltungen der erfindungsgemäßen Vorrichtung zum Bereitstellen von therapeutischem Sauerstoff beschrieben. Diese Ausgestaltungen gelten allerdings ebenso für das System zum Bereitstellen von Notfallsauerstoff sowie therapeutischem Sauerstoff für das Flugzeug aufweisend ein System zum Bereitstellen von Notfallsauerstoff sowie therapeutischem Sauerstoff und die Verwendung eines Systems zum Bereitstellen von Notfallsauerstoff sowie therapeutischem Sauerstoff in einem Flugzeug.

Gemäß einem exemplarischen Ausführungsbeispiel der vorliegenden Erfindung weist die Vorrichtung zum Bereitstellen von therapeutischem Sauerstoff ferner einen zweiten Anschluss für eine Applikationsvorrichtung auf, welcher zweite Anschluss für eine zweite vorbestimmte Durchflussrate ausgelegt ist.

Auch hier mag die zweite vorbestimmte Durchflussrate im Wesentlichen gleich der ersten vorbestimmten Durchflussrate oder aber von dieser verschieden sein. Somit mag eine zustandsabhängige Applikation von zwei unterschiedlichen Durchflussraten möglich sein, oder aber es mag möglich sein, zwei verschiedene Passagiere gleichzeitig mit derselben oder auch unterschiedlichen Durchflussraten mit therapeutischem Sauerstoff zu versorgen.

Gemäß einem weiteren exemplarischen Ausführungsbeispiel der vorliegenden Erfindung mag zumindest einer des ersten Anschlusses und des zweiten Anschlusses autoaktivierend ausgebildet sein.

Autoaktivierend mag auch hier als das automatische Aktivieren durch Anstecken einer Applikationsvorrichtung an einen Anschluss und somit Freischalten des Sauerstoffflusses verstanden werden.

Gemäß einem weiteren exemplarischen Ausführungsbeispiel der vorliegenden Erfindung mag die Vorrichtung zum Bereitstellen von therapeutischem Sauerstoff eingerichtet sein, in einem Personal Supply Channel eines Flugzeugs eingebracht zu werden.

Hierdurch mag eine optisch nicht invasive bzw. im nicht benötigten Zustand unsichtbare Platzierung der Vorrichtung zum Bereitstellen von therapeutischem Sauerstoff innerhalb der Flugzeugkabine möglich sein.

Gemäß einem weiteren exemplarischen Ausführungsbeispiel der vorliegenden Erfindung mag die Vorrichtung zum Bereitstellen von therapeutischem Sauerstoff eine Regelvorrichtung zum Einstellen einer vorbestimmten Sauerstoffflussrate aufweisen.

Die Regelvorrichtung mag hierbei auf zumindest einen des ersten Anschlusses und des zweiten Anschlusses wirken, auf beide gleichsam oder aber, abhängig von der jeweils vorbestimmten Durchflussrate der einzelnen Anschlüsse, unterschiedlich wirken. Auch mag ein unabhängiges Einstellen einer vorbestimmten Sauerstoffflussrate für den jeweiligen ersten bzw. zweiten Anschluss möglich sein. Gemäß einem weiteren exemplarischen Ausführungsbeispiel der Erfindung mag die Vorrichtung zum Bereitstellen von therapeutischem Sauerstoff derart eingerichtet sein, um in dem ersten Betriebszustand an dem zumindest einen Anschluss eine vorbestimmte Notfallsauerstoffflussrate bereitzustellen.

Diese Notfallsauerstoffflussrate mag abhängig von einem erhöhten Versorgungsdruck im Sauerstoffleitungssystem im ersten Betriebszustand sein und mag darüber hinaus auch noch zusätzlich von der ersten bzw. zweiten vorbestimmten Durchflussrate abhängig sein, insbesondere um diese über die reguläre Notfallsauerstoffflussrate erhöht sein.

Ebenfalls mag eine weiterführende Regelmöglichkeit unter Verwendung der Regelvorrichtung zum Einstellen einer vorbestimmten Sauerstoffflussrate denkbar sein, wobei jedoch die vorbestimmte Notfallsauerstoffflussrate, wie sie durch die Vorrichtung zum Bereitstellen von Notfallsauerstoff zur Verfügung gestellt wird, nicht unterschritten werden mag.

Gemäß einem weiteren exemplarischen Ausführungsbeispiel der Erfindung mag die Vorrichtung zum Bereitstellen von therapeutischem Sauerstoff weiterhin eine Vorrichtung zum Bereitstellen von Notfall-Sauerstoff aufweisen, wobei die Vorrichtung zum Bereitstellen von Notfall-Sauerstoff einen zweiten Sauerstoffeingang aufweist, der mit dem ersten Sauerstoffausgang ver- bzw. gekoppelt ist.

Somit lässt sich eine kompakte, jedoch noch immer modulare Vorrichtung schaffen, die in den Personal Supply Channel eines Flugzeuges als Einheit eingebracht werden kann. Somit mag ein Bereitstellen von Notfallsauerstoff sowie therapeutischem Sauerstoff an jedem Sitzplatz im Flugzeug ermöglicht werden.

Ausführungsbeispiele der Erfindung sind in den nachfolgenden Figuren dargestellt und werden im Weiteren näher erläutert.

Es zeigen:
- Figur 1: eine schematische Darstellung des Systems zum Bereitstellen von Notfallsauerstoff sowie therapeutischem Sauerstoff,
- Figur 2: eine Rückansicht der Vorrichtung zum Bereitstellen von therapeutischem Sauerstoff,
- Figur 3: eine Vorderansicht der Vorrichtung zum Bereitstellen von therapeutischem Sauerstoff,
- Figur 4: eine kombinierte Ansicht der Vorrichtung zum Bereitstellen von therapeutischem Sauerstoff sowie der Vorrichtung zum Bereitstellen von Notfallsauerstoff zur Anordnung im PSC.

Gleiche oder ähnliche Komponenten in unterschiedlichen Figuren sind mit gleichen Bezugsziffern versehen.

Die Darstellungen in den Figuren sind schematisch und nicht maßstäblich.

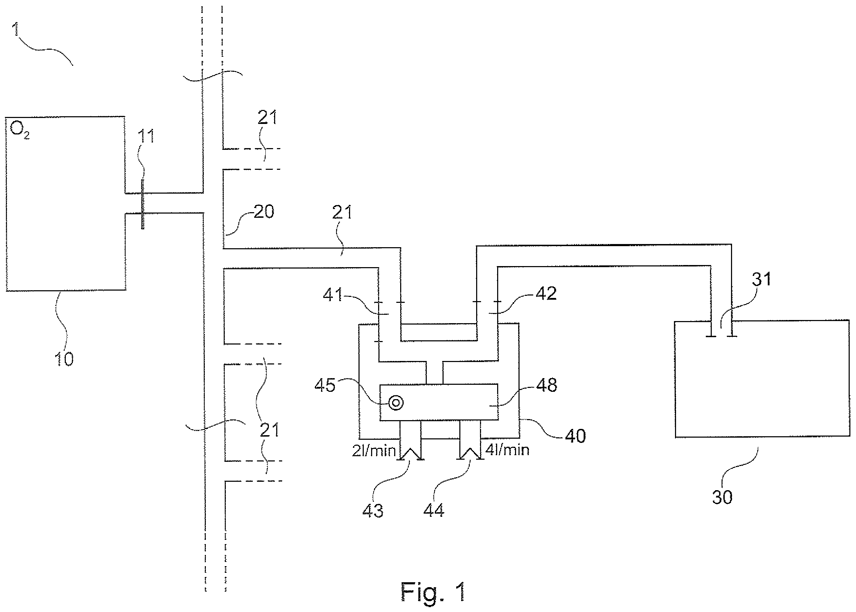

Im Weiteren wird Bezug nehmend auf Figur 1 ein System zum Bereitstellen von Notfallsauerstoff sowie therapeutischem Sauerstoff gemäß einem exemplarischen Ausführungsbeispiel der vorliegenden Erfindung beschrieben.

Das System zum Bereitstellen von Notfallsauerstoff sowie therapeutischem Sauerstoff 1 weist eine Sauerstoffquelle 10 auf, an welche das Sauerstoffleitungssystem 20 angeschlossen ist, welches im Weiteren im Flugzeug verteilt angeordnet ist.

Das Sauerstoffleitungssystem 20 verzweigt hierzu im Flugzeug und weist verteilte zweite Sauerstoffausgänge 21 auf. Aktivieren bzw. Deaktivieren des zweiten Betriebszustandes erfolgt über die hier nur schematisch gezeichnete Vorrichtung 11. Die Vorrichtung 11 mag manuell vom Flugzeugpersonal an einer zentralen Stelle bedient werden oder aber mag mit den Anschlüssen 43, 44 gekoppelt sein.

Der zweite Sauerstoffausgang 21 ist mit dem ersten Sauerstoffeingang 41 der Vorrichtung zum Bereitstellen von therapeutischem Sauerstoff 40 verkoppelt.

Die Vorrichtung zum Bereitstellen von therapeutischem Sauerstoff 40 weist im Weiteren einen ersten Sauerstoffausgang 42 auf, welcher mit dem zweiten Sauerstoffeingang 31 der Vorrichtung zum Bereitstellen von Notfallsauerstoff 30 verkoppelt ist.

Hierbei wird der erste Sauerstoffeingang 41 zum ersten Sauerstoffausgang 42 durchgeschleift und zweigt darüber hinaus zu den beiden Anschlüssen 43 und 44 ab. Die Abzweigung, Aufteilung bzw. Verteilung und insbesondere die Maßnahmen zum Sicherstellen der jeweiligen vorbestimmten Durchflussrate der Anschlüsse 43, 44 sind in Figur 1 nicht näher, insbesondere nur als schematische Verteilvorrichtung 48, dargestellt.

Das Durchschleifen mag neben der in Figur 1 dargestellten Möglichkeit auch in der Verteilvorrichtung 48 als deren Bestandteil erfolgen.

Die Vorrichtung zum Bereitstellen von therapeutischem Sauerstoff 40 weist weiterhin eine Regel- bzw. Steuervorrichtung 45 auf, die ein Einstellen der ersten bzw. zweiten vorbestimmten Durchflussrate gemeinsam oder individuell ermöglicht.

Hierbei mag die Durchflussrate manuell, beispielsweise durch ein Stellrad, eingestellt werden, oder aber es mag auch eine automatische Durchflussregelung auf einen eingestellten Sollwert erfolgen, beispielsweise unter Verwendung eines Druckminderers.

Die Anschlüsse 43, 44 sind eingerichtet, jeweils eine Applikationsvorrichtung wie zum Beispiel eine medizinische Sauerstoffmaske anzuschließen. In Figur 1 ist hierzu exemplarisch der Anschluss 43 als ein Anschluss mit einer vorbestimmten Durchflussrate von zwei Litern Sauerstoff je Minute und der Anschluss 44 mit einer zweiten vorbestimmten Durchflussrate von vier Litern Sauerstoff je Minute dargestellt

Diese Angabe mag im Weiteren eine jeweilige minimale vorbestimmte Durchflussrate der Anschlüsse 43, 44 darstellen, welche mittels der Regelvorrichtung 45 bis zu einem maximal möglichen Wert von beispielsweise sechs Litern Sauerstoff pro Minute erhöht werden kann (individuell oder gemeinschaftlich). Andere vorbestimmte Durchflussraten sind denkbar.

In Figur 1 nicht dargestellt, jedoch durchführbar, ist der direkte Anschluss des zweiten Sauerstoffausgangs 21 an dem zweiten Sauerstoffeingang 31, wodurch es möglich ist, die Vorrichtung zum Bereitstellen von therapeutischem Sauerstoff aus dem Sauerstoffleitungssystem vollständig auszukoppeln.

Weiterhin sind in Figur 1 nicht dargestellte Rückschlagventile im Sauerstoffleitungssystem 20 denkbar.

Sowohl die Vorrichtung zum Bereitstellen von Notfallsauerstoff 30 als auch die Vorrichtung zum Bereitstellen von therapeutischem Sauerstoff 40 ist, in Figur 1 nicht dargestellt, in einem Personal Supply Channel (PSC) einbringbar.

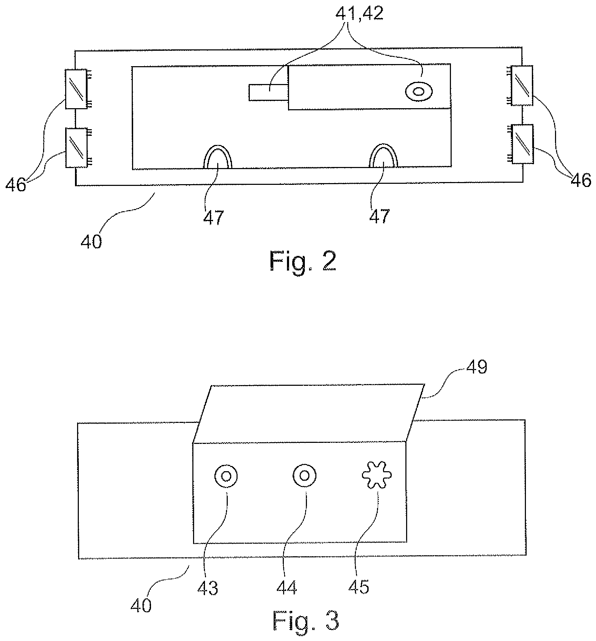

Weiter Bezug nehmend auf Figur 2 ist die Rückansicht einer Vorrichtung zum Bereitstellen von therapeutischem Sauerstoff 40 dargestellt.

Die Vorrichtung weist hierbei erst einen ersten Sauerstoffeingang 41 sowie einen ersten Sauerstoffausgang 42 auf, welche hier als pneumatische Anschlüsse ausgeführt sein mögen.

Die pneumatischen Anschlüsse mögen hierbei als druckdichte Schnellkoppelanschlüsse ausgeführt sein, jedoch ist auch eine permanente Verbindung, beispielsweise per Schlauchschelle, denkbar.

Die Vorrichtung 40 weist weiterhin Schlauchhalter 47 auf, um ein einfaches und knickfreies Führen bzw. Verlegen des Sauerstoffleitungssystems, hier beispielsweise druckfeste Schläuche, zu ermöglichen.

Weiterhin weist die Vorrichtung zum Bereitstellen von therapeutischem Sauerstoff 40 Schnellmontageelemente 46 auf, wie sie üblicherweise bei Vorrichtungen die in den PSC eingebracht werden, Verwendung findet.

Hierdurch mag eine schnelle bzw. kurzfristige Montage und Demontage der Vorrichtung 40 möglich sein, einerseits um diese beispielsweise während der Standzeit eines Flugzeuges nachzurüsten, oder aber diese im Servicefall leicht und schnell ausbauen zu mögen.

Bezug nehmend auf Figur 3 wird die Vorderansicht der erfindungsgemäßen Vorrichtung zum Bereitstellen von therapeutischem Sauerstoff 40 dargestellt.

Die Vorrichtung 40 weist hierbei eine Sichtklappe 49 auf, welche in geschlossenem Zustand die einzelnen Elemente der Vorrichtung zum Bereitstellen von therapeutischem Sauerstoff 40 optisch verdeckt. Die Klappe mag mit einem nicht dargestellten Mechanismus versehen sein, um ein Öffnen und Schließen nur durch das Flugzeugpersonal sicherzustellen. Hierzu mag beispielsweise ein spezieller Schlüssel verwendet werden oder aber der Mechanismus mag verdeckt angeordnet sein.

Unter der Klappe 49 befinden sich in Figur 3 dargestellt Anschlüsse 43, 44 zum Anschluss jeweils einer Applikationsvorrichtung, wie zum Beispiel einer medizinischen Sauerstoffmaske, sowie die Regelvorrichtung zum Einstellen einer vorbestimmten Durchflussrate. Die jeweils eingestellte vorbestimmte Durchflussrate mag über nicht dargestellte Markierungen visualisiert werden, oder aber es ist auch die Verwendung einer separaten, hier nicht dargestellten Anzeige denkbar.

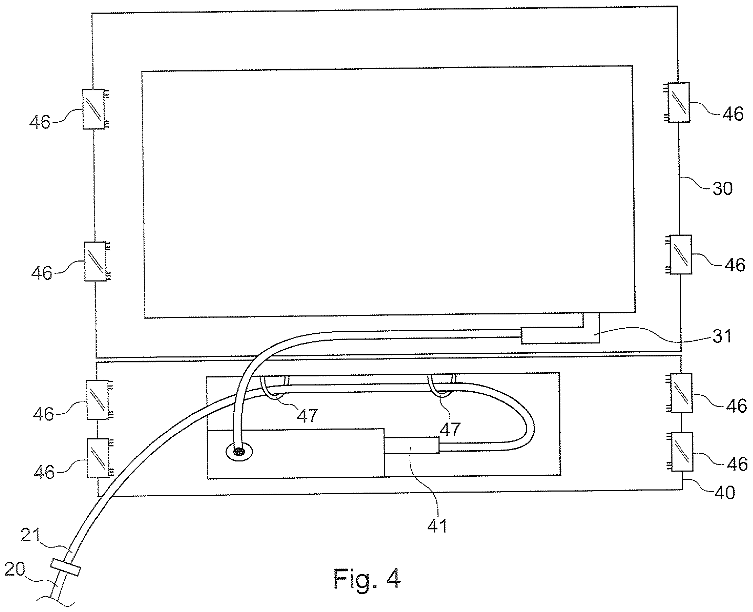

In Figur 4 ist die rückwärtige Ansicht der Kombination einer Vorrichtung zum Bereitstellen von Notfallsauerstoff 30 mit einer Vorrichtung zum Bereitstellen von therapeutischem Sauerstoff 40 dargestellt.

Der zweite Sauerstoffausgang 21 des Sauerstoffleitungssystems 20 wird, hier als Schlauch gehalten durch die beiden Schlauchhalter 47, zum ersten Sauerstoffeingang 41 geführt. Im Weiteren wird der erste Sauerstoffausgang 42 mit dem zweiten Sauerstoffeingang 31 der Vorrichtung zum Bereitstellen von Notfallsauerstoff 30 verkoppelt.

Beide Vorrichtungen 30, 40 werden mittels der Schnellmontageelemente 46 im PSC angebracht. Eine mögliche bzw. übliche Breite der Seite, an der die Schnellmontageelemente 46 angebracht sind, ist für die Vorrichtung zum Bereitstellen von therapeutischem Sauerstoff 40 drei Zoll.

Die Erfindung beschränkt sich in ihren Ausführungen nicht auf die in den Figuren dargestellten, bevorzugten Ausführungsformen. Vielmehr sind eine Vielzahl von Varianten denkbar, welche von der dargestellten Lösung und dem erfindungsgemäßen Prinzip auch bei grundsätzlich anders gearteten Ausführungsformen Gebrauch machen.

Es sei darauf hingewiesen, dass das erfindungsgemäße Prinzip nicht auf Flugzeuge beschränkt ist, sondern vielmehr in beliebigen Verkehrsmitteln, insbesondere bei Zügen, Schiffen, Omnibussen etc. Anwendung finden kann.

Ergänzend sei darauf hingewiesen, dass "aufweisend" bzw. "umfassend" keine anderen Elemente oder Schritte ausschließen und dass "eine" oder "ein" keine Vielzahl ausschließen. Ferner sei darauf hingewiesen, dass Merkmale, die mit Verweis auf eines der obigen Ausführungsbeispiele beschrieben worden sind, auch in Kombination mit anderen Merkmalen anderer oben beschriebener Ausführungsbeispiele verwendet werden können. Bezugszeichen in den Ansprüchen sind nicht als Einschränkungen anzusehen.

### Bezugszeichenliste

- 1: System zum Bereitstellen von Notfallsauerstoff sowie therapeutischem Sauerstoff

- 10: Sauerstoffquelle
- 11: Vorrichtung zum Aktivieren/Deaktivieren des zweiten Betriebszustandes

- 20: Sauerstoffleitungssystem
- 21: Zweiter Sauerstoffausgang

- 30: Vorrichtung zum Bereitstellen von Notfallsauerstoff
- 31: Zweiter Sauerstoffeingang

- 40: Vorrichtung zum Bereitstellen von therapeutischem Sauerstoff
- 41: Erster Sauerstoffeingang
- 42: Erster Sauerstoffausgang
- 43: Erster Anschluss
- 44: Zweiter Anschluss
- 45: Regelvorrichtung
- 46: Schnellmontageelement
- 47: Schlauchhalter
- 48: Verteilvorrichtung
- 49: Sichtklappe

## Patentansprüche

1. Vorrichtung zum Bereitstellen von therapeutischem Sauerstoff (40), aufweisend:
einen ersten Sauerstoffeingang (41); und
einen ersten Sauerstoffausgang (42);
wobei ein zweiter Sauerstoffausgang (21) eines Sauerstoff-Leitungssystems (20) mit dem ersten Sauerstoffeingang (41) koppelbar ist,
der erste Sauerstoffausgang (42) mit einem zweiten Sauerstoffeingang (31) einer Vorrichtung zum Bereitstellen von Notfallsauerstoff (30) koppelbar ist, wobei der erste Sauerstoffeingang (41) intern zu dem ersten Sauerstoffausgang (42) durchgeschleift ist, wobei
die Vorrichtung zum Bereitstellen von therapeutischem Sauerstoff (40) einen ersten Anschluss (43) für eine Applikationsvorrichtung aufweist, wobei der erste Anschluss (43) für eine erste vorbestimmte Durchflussrate ausgelegt ist;
wobei die Vorrichtung zum Bereitstellen von therapeutischem Sauerstoff (40) ferner einen zweiten Anschluss (44) für eine Applikationsvorrichtung aufweist, wobei der zweite Anschluss (44) für eine zweite vorbestimmte Durchflussrate ausgelegt ist, die sich von der ersten vorbestimmten Durchflussrate unterscheidet.

2. Vorrichtung nach Anspruch 1, wobei
die Vorrichtung derart eingerichtet ist, um in einem ersten Betriebszustand an dem ersten Anschluss (43) oder an dem zweiten Anschluss (44) eine vorbestimmte Notfall-Sauerstoff-Flussrate bereit zu stellen.

3. System zum Bereitstellen von Notfallsauerstoff sowie therapeutischem Sauerstoff in einem Flugzeug, aufweisend:
eine Vorrichtung gemäß einem der Ansprüche 1 oder 2;
eine Sauerstoffquelle (10);
wobei das Sauerstoff-Leitungssystem (20) den zweiten Sauerstoffausgang (21) aufweist;
wobei die Vorrichtung zum Bereitstellen von Notfallsauerstoff (30) den zweiten Sauerstoffeingang (31) aufweist;
wobei der zweite Sauerstoffausgang (21) mit dem ersten Sauerstoffeingang (41) koppelbar ist,
wobei der erste Sauerstoffausgang (42) mit dem zweiten Sauerstoffeingang (31) koppelbar ist, und
der zweite Sauerstoffausgang (21) mit dem zweiten Sauerstoffeingang (31) koppelbar ist.

4. System nach Anspruch 3, wobei zumindest einer des ersten Anschlusses (43) und des zweiten Anschlusses (44) autoaktivierend ausgebildet ist.

5. System nach Anspruch 3 oder 4, wobei zumindest eine der Vorrichtung zum Bereitstellen von Notfallsauerstoff (30) und der Vorrichtung zum Bereitstellen von therapeutischem Sauerstoff (40) eingerichtet ist, in einem Personal Supply Channel (3) eines Flugzeuges (2) eingebracht zu werden.

6. System nach einem der Ansprüche 3 bis 5, wobei
der Sauerstoff-Betriebsdruck im Sauerstoff-Leitungssystem (20) in einem zweiten Betriebszustand zur Applikation von therapeutischem Sauerstoff geringer ist als in einem ersten Betriebszustand zur Applikation von Notfall-Sauerstoff, insbesondere geringer als ein Auslösedruck, mit dem die Vorrichtung zum Bereitstellen von Notfallsauerstoff (30) aktivierbar ist.

7. System nach einem der Ansprüche 3 bis 6, wobei
die Vorrichtung zum Bereitstellen von therapeutischem Sauerstoff (40) eine Regel- bzw. Steuervorrichtung (45) zum Einstellen einer vorbestimmten Sauerstoff-Flussrate aufweist.

8. System nach Anspruch 6, weiterhin aufweisend
eine Vorrichtung (11) zum Aktivieren bzw. Deaktivieren des zweiten Betriebszustandes.

9. Ein Flugzeug, aufweisend ein System nach einem der Ansprüche 3 bis 8.

## Claims

1. A device for providing therapeutic oxygen (40), comprising:
a first oxygen inlet (41); and
a first oxygen outlet (42);
wherein a second oxygen outlet (21) of an oxygen conduction system (20) is adapted to be coupled with the first oxygen inlet (41), the first oxygen outlet (42) is adapted to be coupled with a second oxygen inlet (31) of a device for providing emergency oxygen (30); wherein the first oxygen inlet (41) is internally looped through to the first oxygen outlet (42); wherein the device for providing therapeutic oxygen (40) has a first port (43) for an application device, wherein the first port (43) is designed for a predetermined flow rate;
wherein the device for providing therapeutic oxygen (40) further comprises a second port (44) for an application device,
wherein the second port (44) is adapted for a second predetermined flow rate which is different from the first predetermined flow rate.

2. The device of claim 1, wherein the device is set up in such a way as to provide a predetermined emergency oxygen flow rate in a first operating state at the first port (43) or at the second port (44).

3. A system for providing emergency oxygen and therapeutic oxygen in an aircraft, comprising:
a device according to one of claims 1 or 2;
an oxygen source (10);
wherein the oxygen conduction system (20) comprises the second oxygen outlet (21); wherein the device for providing emergency oxygen (30) comprises the second oxygen inlet (31);
wherein the second oxygen outlet (21) is adapted to be coupled with the first oxygen inlet (41), the first oxygen outlet (42) is adapted to be coupled with the second oxygen inlet (31), and the second oxygen outlet (21) is adapted to be coupled with the second oxygen inlet (31).

4. The system of claim 3, wherein at least one of the first port (43) or the second port is auto-activating (44).

5. The system of one of claims 3 or 4, wherein at least one of the device for providing emergency oxygen (30) or the device for providing therapeutic oxygen (40) is designed to be incorporated in a personal supply channel (3) of an aircraft (2).

6. The system of one of claims 3 to 5, wherein a working pressure of oxygen in the oxygen conduction system (20) is lower in a second operating state for the application of therapeutic oxygen than in a first operating state for the application of emergency oxygen, in particular lower than a triggering pressure, at which the device for providing emergency oxygen (30) is activated.

7. The system of one of claims 3 to 6, wherein the device for providing therapeutic oxygen (40) has a regulator or controller (45) for setting a predetermined oxygen flow rate.

8. The system of claim 6, further comprising
a device (11) for activating or deactivating the second operating state.

9. An aircraft comprising a system according to one of claims 3 to 8.

## Revendications

1. Dispositif pour fournir de l'oxygène thérapeutique (40), comprenant:
une première entrée d'oxygène (41); et
une première sortie d'oxygène (42);
dans lequel une seconde sortie d'oxygène (21) d'un système de conduite d'oxygène (20) peut être couplée à la première entrée d'oxygène (41),
la première sortie d'oxygène (42) peut être couplée à une seconde entrée d'oxygène (31) d'un dispositif d'alimentation en oxygène d'urgence (30), dans lequel
la première entrée d'oxygène (41) est bouclée intérieurement jusqu'à la première sortie d'oxygène (42), dans lequel
le dispositif pour fournir de l'oxygène thérapeutique (40) comprend une première connexion (43) pour un dispositif d'application, dans laquelle la première connexion (43) est configuré pour un premier débit prédéterminé; dans lequel le dispositif pour fournir de l'oxygène thérapeutique (40) comprend en outre un seconde connexion (44) pour un dispositif d'application, dans lequel la seconde connexion (44) est configuré pour un second débit prédéterminé différent du premier débit prédéterminé.

2. Dispositif selon la revendication 1, dans lequel
le dispositif est agencé pour fournir un débit d'oxygène d'urgence prédéterminé à la première connexion (43) ou à la seconde connexion (44) dans une première condition de fonctionnement.

3. Système pour fournir de l'oxygène d'urgence aussi bien que de l'oxygène thérapeutique dans un aéronef, comprenant:
un dispositif selon l'une des revendications 1 ou 2; une source d'oxygène (10);
dans lequel le système de conduit d'oxygène (20) comprend la deuxième sortie d'oxygène (21);
dans lequel le dispositif pour fournir de l'oxygène d'urgence (30) comprend la seconde entrée d'oxygène (31);
dans lequel la seconde sortie d'oxygène (21) peut être couplée à la première entrée d'oxygène (41),
dans lequel la première sortie d'oxygène (42) peut être couplée à la seconde entrée d'oxygène (31), et
la seconde sortie d'oxygène (21) peut être couplée à la seconde entrée d'oxygène (31).

4. Système selon la revendication 3, dans lequel au moins l'un parmi la première connexion (43) et la seconde connexion (44) est adapté de manière auto-activé.

5. Système selon la revendication 3 ou 4, dans lequel au moins l'un du dispositif pour fournir de l'oxygène d'urgence (30) et du dispositif pour fournir de l'oxygène thérapeutique (40) est conçu pour être déployé dans un Personal Supply Channel (3) d'un aéronef.

6. Système selon l'une quelconque des revendications 3 à 5, dans lequel la pression d'oxygène d'exploitation dans le système de conduite d'oxygène (20) dans un second état de fonctionnement pour l'application d'oxygène thérapeutique est inférieure à un premier état de fonctionnement pour l'application d'oxygène d'urgence, en particulier inférieure à une pression de déclenchement, avec laquelle le dispositif pour fournir de l'oxygène d'urgence (30) est activable.

7. Système selon l'une quelconque des revendications 3 à 6, dans lequel le dispositif pour fournir de l'oxygène thérapeutique (40) comprend un dispositif de régulation ou de commande (45) pour régler un débit d'oxygène prédéterminé.

8. Système selon la revendication 6, comprenant en outre un dispositif (11) pour activer ou désactiver le second état de fonctionnement.

9. Avion comprenant un système selon l'une quelconque des revendications 3 à 8.
